# FASCICULE DE BREVET EUROPEEN

(11) **EP 0 515 237 B1**
(45) Date de publication et mention de la délivrance du brevet: **17.07.1996**
(21) Numéro de dépôt: 92401009.3
(22) Date de dépôt: 10.04.1992
(51) Int. Cl.: C12N 1/04, C12N 3/00, A23C 19/032

(54) **Composition appropriée à la conservation de spores fongiques activées**
Zusammensetzung, geeignet zur Konservierung von aktiven Pilz-Sporen
Composition suitable for the preservation of active fungal spores

(30) Priorité: 24.05.1991 FR 9106263
(43) Date de publication de la demande: 25.11.1992
(73) Titulaire: Société TEXEL, F-86220 Dange Saint Romain (FR)
(72) Inventeur: Chataud, Jean, F-37800 Sainte Maure de Touraine (FR); Charlot, Marc, F-86100 Chatellerault (FR)
(74) Mandataire: Seugnet, Jean Louis

(56) Documents cités:
- FR-A- 1 338 983
- FR-A- 1 596 471
- FR-A- 2 346 445
- FR-A- 2 600 671
- US-A- 4 021 581
- US-A- 4 226 940
- Dictionnaire de la chimie et de ses applications, pages 49-50, 75, 397. 579, 624, 629, 773, 917 (1959)
- Concise chemical and technical dictionary, 1974, H. Bennett, pages 542, 835, 837

## Description

La présente invention concerne une composition à base de microorganismes et plus particulièrement de moisissures sous forme de spores prégermées et/ou spores germées, appropriée à la conservation prolongée desdits microorganismes.

Les moisissures sont des champignons dont l'appareil végétatif ou thalle est pourvu d'une paroi. Ce sont des hétérotrophes qui se nourissent par absorption. Ils peuvent se présenter sous la forme de spores ou conidies qui assurent la reproduction assexuée desdites moisisssures.

Au cours de leur évolution métabolique les spores peuvent germer et conduire ainsi à des spores prégermées et/ou spores germées. On entend par spores pré-germées des spores qui présentent une croissance sphérique (renflement) et qui n'ont pas encore développé un bourgeon germinatif et spores germées des spores comportant au moins un tube germinatif.

Les champignons tels les moisissures à l'état non germinatif, sont utilisés notamment en tant que ferment dans l'industrie alimentaire, par exemple en fromagerie pour transformer des produits comme le lait en fromage et en salaison pour la fabrication du saucisson.

Ces moisissures sont généralement commercialisées sous forme de spores, soit en milieu liquide, soit sous une forme congelée ou sous forme de poudres lyophilisées.

FR-A 1 338 983 décrit un procédé de conservation de spores de moisissures, comme par exemple des spores de pénicillium, caractérisé en ce que l'on prépare une composition comprenant les spores, des protéines (comme des protéines de lait ou des allumines) et des glucides (comme du lactose ou du glucose) et que l'on refroidit l'ensemble.

Les spores sont très résistantes et présentent l'avantage d'une excellente stabilité dans le temps. Lors de leur utilisation les spores sont généralement pulvérisées et/ou incorporées dans les produits à transformer.
Les spores germent et se développent sous forme d'une couverture mycélienne.

Toutefois, pendant la période de formation du mycélium à partir des spores, il existe un risque important de contamination des produits à transformer, par des microorganismes indésirés.
De plus, cette période nécessaire au développement de la moisissure correspond à une perte économique pour l'utilisateur due à une commercialisation retardée et un investissement important de stockage des produits à transformer.

De manière à diminuer cette période de formation du mycélium, on peut envisager l'emploi direct des spores prégermées et/ou spores germées.

Toutefois, l'utilisation et la commercialisation de ce type de spores beaucoup moins stables et résistantes que les spores d'origine, ont été jusqu'ici limitées par le fait que l'on ne connaissait pas de moyen pour les consever pendant une longue période de temps, sans les dégrader et sans qu'elles perdent leur activité.

Un premier objet de l'invention consiste donc en une composition permettant notamment une longue conservation de moisissures, lorsqu'elles se présentent sous forme de spores prégermées et/ou spores germées.

Un deuxième objet de l'invention est l'utilisation dans l'industrie alimentaire de ladite composition.

Un dernier objet de l'invention est un procédé de conservation desdites spores de moisissures mettant en oeuvre ladite composition.

La présente invention concerne une composition à base de spores fongiques activées choisies parmi des spores prégermées et/ou des spores germées susceptibles d'être obtenues par inoculation d'un milieu de culture ou d'un milieu de germation, caractérisée en ce que ladite composition comporte une phase aqueuse contenant au moins un polyalcool, au moins un composé protéique et lesdites spores fongiques activées.

On entend désigner de manière générale, par spore fongiques activées, des spores de moisissures prégermées et/ou des spores de moisissures germées.

Toutefois, la présente invention s'applique plus particulièrement à la conservation des spores dites pré-germées.

La concentration en microorganismes de la composition selon l'invention varie en fonction de l'utilisation ultérieure. En général la composition comporte plus de 10⁶ microorganismes/ml et de préférence de 10⁷ à 10¹⁰ microorganismes/ml.

Les moisissures préferées sont celles du genre Penicillium et Geotrichum. Parmi les Penicillium, on utilise plus particulièrement en fromagerie P. camenbertii, P. caseicolum et P. roquefortii. En salaison, on utilise plus particulièrement les espèces P. nalgiovensis, P. chrysogenum et, dans une moindre mesure P. camembertii et P. caseicolum.

La phase aqueuse peut-être constituée par un soluté aqueux comme de l'eau de ville ou une solution aqueuse de chlorure de sodium, et/ou par un milieu de culture des microorganismes, ces derniers pouvant être préalablement concentrés ou non, par exemple par décantation ou centrifugation du milieu de culture. De tels milieux de culture sont définis ci-dessous.

Ledit composé protéique peut être une protéine entière, hydrolysée ou partiellement hydrolysée telles que les protéines du lait comme les lactalbumines ou les caséines, les protéines de l'oeuf comme l'ovalbumine, les protéines de poissons, les protéines d'origine végétale comme les protéines de soja, la serumalbumine ou la gélatine. Ledit compose protéique peut encore être une glycoprotéine ou un milieu biologique comportant au moins une protéine et/ou une glycoprotéine tels l'oeuf entier, le blanc d'oeuf commercial, montant ou non, les farines de poisson ou les farines d'origine végétale comme les farines de soja, le lait entier, dégraissé, partiellement dégraissé ou en poudre.

Le polyalcool peut être un hexitol comme le sorbitol ou le mannitol, ou avantageusement le glycérol.

La composition selon l'invention peut également comporter un glucide qui peut être un ose tels le glucose, le fructose, le galactose ou un oside tels le lactose, le maltose, le saccharose, ou les amidons, les maltodextrines et les dextrines, les sirops de glucose hydrogénés.

Généralement, on peut obvier dudit glucide lorsque dans la composition on utilise à titre de compose protéique une glycoprotéine ou un milieu biologique comportant une glycoprotéine ou une protéine ainsi qu'un glucide. De tels milieux biologiques sont par exemple le lait ou l'oeuf entier.

La composition selon l'invention est généralement constituée de 60 à 95 % en poids, de préférence de 70 à 90 % en poids de ladite phase aqueuse, de 5 à 40 % en poids, de préférence de 10 à 30 % en poids, dudit composé protéique, de 0,5 à 4 % en poids, de préférence de 1 à 3 % en poids dudit polyalcool et d'une manière avantageuse de 5 à 45 % en poids, de préférence de 10 à 30 % d'un composé glucidique.

La composition de l'invention peut comporter d'une manière avantageuse au moins un agent antioxydant. A titre d'agent antioxidant on peut citer les acides citrique, ascorbique et glutamique ainsi que leurs sels alcalins ou alcalino-terreux, en particulier les sels de sodium, potassium, calcium et magnésium. On utilise generalement de 0,1 à 2 % en poids, de préférence de 0,4 à 1 % en poids dudit agent antioxydant par rapport au poids de la composition.

Selon un mode de réalisation particulier de l'invention, la composition comporte en outre un compose hydrophobe et se présente alors sous une forme d'émulsion. Il s'agit d'une émulsion eau dans l'huile, stable dans le temps et supportant la congélation.

A titre de compose hydrophobe avantageux dans le cadre de la présente invention, on peut citer les huiles telles que les huiles d'origine animale comme l'huile de foie de morue, ou les huiles d'origine végétale comme les huiles de soja, de colza, d'olive, d'arachide, de maïs ou de tournesol.

En présence de ce composé hydrophobe, la composition selon l'invention peut contenir indifféremment à titre de spores activées, des spores prégermées et/ou spores germées.

En particulier la présente invention se rapporte également à une composition constituée de 15 à 50 % en poids, de préférence de 25 à 41 % en poids de ladite phase aqueuse, de 35 à 80 % en poids, de préférence de 50 à 70 % en poids dudit compose hydrophobe, de 2 à 8 % en poids, de préférence de 3 à 6 % en poids d'au moins un composé protéique,de 0,5 à 4 % en poids, de préférence de 1 à 3 % en poids d'au moins un polyalcool et de 0 à 3 % en poids d'au moins un glucide.

Les spores activées utilisées selon la présente invention peuvent être préparées par inoculation d'un milieu de culture (ou milieu de germination) par des spores. Le milieu de culture peut comporter outre de l'eau, une source azotée, une source carbonée, des ions phosphates, et éventuellement des oligoéléments.

La source azotée peut consister en une ou plusieurs protéines ou en un hydrolysat total ou partiel de protéines telles celles décrites ci-dessus, en un ou plusieurs acides amines, en un lysat ou un autolysat de levures et/ou en une source d'azote minérale comme les sels d'ammonium ou les nitrates par exemple le nitrate de potassium ou le nitrate de sodium.

La source carbonée peut être constituée par au moins un glucide comme le glucose, le saccharose, le fructose, le lactose, les amidons, les dextrines ou les maltodextrines ou par un hexitol.

Les ions phosphates peuvent être apportés par un sel monobasique de potassium ou de sodium, tels les dihydrogénophosphates de potassium ou de sodium.

Le milieu de culture peut également comporter des oligoéléments tels le cuivre, le manganèse, le fer, le cobalt, le zinc ou le magnésium. Ces oligoéléments peuvent être apportes sous forme de sulfates.

Généralement le milieu de culture comporte de 0,15 à 0,80 % en poids, de préférence de 0,20 à 0,40 % en poids d'azote amené par la source azotée, de 2 à 15 % en poids, de préférence de 4 à 10 % en poids de la source carbonée et de 5 à 25 mmoles de préférence de 10 à 15 mmoles/l d'ions phosphates.

Le milieu de culture peut également comporter de 0 à 200 mg/l d'oligoéléments. Le pH du milieu de culture est de préférence compris entre 4 et 7, de préférence entre 4 et 6. Le pH dudit milieu peut être ajuste par exemple au moyen d'un acide tels l'acide chlorhydrique, l'acide sulfurique, l'acide nitrique, l'acide orthophosphorique, l'acide lactique ou l'acide tartrique.

La température de germination est généralement comprise entre 10°C et 30°C.

La germination s'effectue sous agitation et sous aération.

La durée de germination peut être comprise entre 8 et 16 heures, plus particulièrement entre 10 et 12 heures. Généralement on introduit dans le milieu de culture une quantité de spores suffisante pour obtenir une concentration finale de spores activées comprise entre 10⁶ et 10¹⁰ /ml. En utilisant un tel milieu de culture, on obtient une quantité de spores activées supérieure à 90 %, et généralement supérieure à 95 %. Le dénombrement des spores peut se faire par comptage à l'hématimètre, ou sur milieux de dénombrement tel que PDA ou MEA.

La présente invention concerne également un procédé de préparation des compositions décrites ci-dessus et qui consiste à mélanger les microorganismes avec ladite phase aqueuse, au moins un compose protéique, au moins un polyalcool et éventuellement au moins un glucide, au moins un agent antioxydant et/ou au moins un compose hydrophobe.

Les microorganismes peuvent être incorporés dans la composition par exemple sous une forme lyophilisée, mais d'une manière avantageuse, pour les spores activées de moisissures la phase aqueuse est constituée du milieu de culture comportant les microorganismes, ce dernier pouvant ou non être préalablement concentré. Si nécessaire, il est possible de compléter la phase aqueuse constituée par le milieu de culture par ajout d'un soluté aqueux tels ceux décrits ci-dessus.

De préférence, on mélange la phase aqueuse contenant les microorganismes avec au moins un composé protéique, au moins un polyalcool et éventuellement au moins un glucide, au moins un agent antioxydant et/ou au moins un composé hydrophobe.

Le mélange est généralement réalisé sous agitation selon des moyens classiques, par exemple une turbine d'agitation. Lorsque la composition selon l'invention contient en outre un composé hydrophobe, l'agitation est adaptée pour permettre la formation d'une émulsion.

Le mélange peut être réalisé à une température comprise entre environ 5 et 30°C et de préférence entre environ 15 et 25°C.

L'invention concerne également un procédé de conservation de ladite composition selon lequel la composition est conservée à une température de + 4°C ou congelée à une température d'environ - 5°C à - 80°C et de préférence entre environ - 5°C et - 30°C.

Un tel procédé permet de conserver des spores activées de moisissures pendant au moins un mois, à + 4°C et généralement pendant au moins 4 mois à - 10°C, sans endommager lesdites spores.

Au moment de l'utilisation des microorganismes, et s'agissant d'une composition congelée, la décongélation ne nécessite aucune précaution particulière, si ce n'est le retour à une température trop élevée, c'est à dire excédant environ 45°C. D'une manière avantageuse la température de décongélation se situe entre 4 et 25 °C. S'agissant d'une préparation conservée à + 4°C, celle-ci peut être utilisée telle quelle.

En présence d'un composé hydrophobe la température de congélation de ladite composition est bien entendu abaissée. Dans ce cas, on congèle la composition à une température d'environ - 30 à - 180°C et de préférence entre environ - 30 et - 80°C. D'une manière avantageuse, on congèle ladite composition en abaissant la température de 0,1°C à 10°C/min et de préférence de 0,1°C à 3°C/min et plus particulièrement de 0,4°C à 1,5°C/min.

Les compositions selon l'invention ayant subi ou non un procédé de conservation, peuvent être utilisées directement ou après dilution à une teneur désirée en microorganismes, dans l'industrie alimentaire, en particulier en fromagerie ou en salaison. Lesdites compositions peuvent par exemple être répandues ou incorporées dans les produits à transformer tels les fromages ou les produits de salaison.

Les exemples donnés ci-après à titre non limitatif permettront de mettre en évidence d'autres avantages et caractéristiques de la présente invention.

### EXEMPLE 1

### a) Germination

Dans un fermenteur on introduit un milieu aqueux stérile à pH 5,1 ajusté à l'acide chlorhydrique et comportant :

| | |
|---|---|
| glucose | 50 g |
| dihydrogénophosphate de potassium | 2 g |
| autolysat de levure | 15 g |
| sulfate de cuivre (5H₂O) | 0,7 mg |
| sulfate de manganèse (H₂O) | 30 mg |
| sulfate de magnésium (7H₂O) | 100 mg |
| eau potable qsp | 1000 ml |

On introduit ensuite dans le fermenteur des spores de Penicillium candidum à raison de 6.10⁸ spores/ml. On place le milieu sous aération et agitation à 21°C pendant 12 heures.

En fin de germination, on obtient un milieu comportant 95 % de spores dites pré-germées.

On concentre ce milieu par simple décantation pendant une heure au moins de manière à obtenir une concentration en spores de 2.10⁹ ml.

### b) Formulation

A l'aide d'une turbine d'agitation, on mélange 500 ml dudit milieu concentré avec :

| | |
|---|---|
| lait en poudre | 32,50 g |
| glycérol | 10,00 g |
| acide ascorbique | 2,50 g |
| glutamate de sodium | 2,50 g |

L'agitation se fait à 1 800 tours/min, à une température de 20°C, pendant 10 minutes.

Après conditionnement de la composition ainsi obtenue, dans des flacons en plastique, on la congèle à une température de - 10°C, ou on la conserve à + 4°C.

Certains flacons sont stockés à + 4°C et les autres à - 10°C.

La stabilité, c'est-à-dire le nombre de spores pré-germées viables par rapport au nombre de spores pré-germées introduites initialement est supérieure à 95 % au bout d'un mois pour la composition stockée à + 4°C, et au bout de 3 mois pour celle stockée à - 10°C.

### EXEMPLE 2

On mélange comme indiqué dans l'exemple 1, une suspension aqueuse de 5 000 ml de spores pré-germées de Penicillium Candidum obtenue dans les mêmes conditions que celles de l'exemple 1 et contenant également 2.10⁹ g de spores pré-germées / ml, avec :

| | |
|---|---|
| glycérol | 83,30 g |
| lait en poudre | 200,00 g |
| blanc d'oeuf montant | 500,00 g |

On introduit la composition dans des flacons, et on congèle la composition à - 10°C. Après 4 mois on la décongèle et on peut constater que la stabilité des spores activées est supérieure à 95 %.

### EXEMPLE 3

Comme indiqué dans l'exemple 1, on mélange 500 ml d'une suspension aqueuse de spores pré-germées de Penicillium obtenue dans les mêmes conditions que celles de l'exemple 1, mais contenant 3.10⁹ spores/ml, avec :

| | |
|---|---|
| glycérol | 8,33 g |
| lait en poudre | 41,66 g |
| blanc d'oeuf montant | 41,66 g |
| acide ascorbique | 2,50 g |
| glutamate de sodium | 2,50 g |

On introduit la composition dans des flacons.

On stocke la composition pendant 4 mois à - 18°C et on la décongèle. La stabilité des spores activées est supérieure à 95 %.

### EXEMPLE 4

Comme indiqué dans l'exemple 1, on mélange 3000 ml d'une suspension aqueuse de spores pré-germées de Penicillium Candidum obtenue dans les mêmes conditions que celles de l'exemple 1, et contenant 2.10⁹ spores/ml avec :

| | |
|---|---|
| glycérol | 50,00 g |
| lait en poudre | 250,00 g |
| blanc d'oeuf non montant | 250,00 g |
| licazym® | 750,00 ml (sirop de glucose hydrogéné). |

On introduit la composition dans des flacons en plastiques qui sont stockés à + 4°C, - 10°C et - 80°C. La stabilité des spores pré-germées est la suivante :
100 % à 1,5 mois de stockage à + 4°C
95 % à 4 mois de stockage à - 10°C
100 % à 4 mois de stockage à - 80°C

### EXEMPLE 5

### a) Germination

On cultive dans un fermenteur comportant le milieu de culture décrit en exemple 1 des spores de Penicillium candidum à raison de 5.10⁷ spores/ml.

En fin de germination le milieu obtenu est concentré de manière à obtenir une concentration en spores activées de 2.10⁸/ml.

### b) Formulation

A l'aide d'une turbine d'agitation, on mélange 500 ml dudit milieu concentré avec :

| | |
|---|---|
| lait en poudre | 18,75 g |
| blanc d'oeuf montant | 62,50 g |
| glycérol | 25,00 g |
| acid ascorbique | 5,00 g |
| glutamate de sodium | 5,00 g |

A ce mélange, on ajoute, toujours sous agitation, 1 000 ml d'huile de tournesol.

L'agitation se fait à 1 800 tours/min, à une température de 20°C, pendant 10 minutes.

Après conditionnement de la composition ainsi obtenue, dans des flacons en plastique, on la congèle à une température de - 35°C suivant une cinétique régulière de descente en température de 0,5°C/min.

Certains flacons congelés sont stockés à - 30°C et les autres à - 80°C. La stabilité, c'est à dire le nombre de spores germées viables après décongélation de la composition par rapport au nombre de spores germées introduites initialement est supérieure à 95 % au bout de 1 mois pour la composition stockée à - 30°C, et au bout de 6 mois pour celle stockée à - 80°C.

### EXEMPLE 6

On mélange comme indiqué dans l'exemple 5, une suspension aqueuse de 5 000 ml de spores germées de Penicillium Candidum obtenue dans les mêmes conditions que celles de l'exemple 5 et contenant également 2.10⁸ spores germées/ml, avec :

| | |
|---|---|
| glycérol | 250 g |
| lait en poudre | 187,5 g |
| blanc d'oeuf montant | 625 g |

Puis on rajoute à ce mélange 9 000 ml d'huile de tournesol et 1 000 ml d'huile de germe de maïs.

On introduit la composition dans des flacons, et on la congèle à - 80°C par abaissement progressif de la température de 1°C par minute. On stocke la composition à - 80°C. Après 6 mois on la décongèle et on peut constater que la stabilité des spores activées est supérieure à 95 %.

### EXEMPLE 7

Comme indiqué dans l'exemple 5, on mélange 500 ml d'une suspension aqueuse de spores germées de Penicillium obtenue dans les mêmes conditions que celles de l'exemple 5, mais contenant 2,5 10⁸ spores/ml, avec :

| | |
|---|---|
| glycérol | 25 g |
| lait en poudre | 18,75 g |
| blanc d'oeuf montant | 62,50 g |
| acide ascorbique | 5 g |
| glutamate de sodium | 5 g |

Puis on rajoute à ce mélange 1.000 ml d'huile de tournesol et on abaisse progressivement la température de 0,5°C/min jusqu'à - 35°C.

On introduit la composition dans des flacons.

On stocke la composition pendant 6 mois à - 80°C et on la décongèle. La stabilité des spores germées est supérieure à 95 %.

## Revendications

1. Composition à base de spores fongiques activées choisies parmi des spores prégermées et/ou des spores germées susceptibles d'être obtenues par inoculation d'un milieu de culture ou d'un milieu de germation, caractérisée en ce que ladite composition comporte une phase aqueuse contenant au moins un polyalcool, au moins un composé protéique et lesdites spores fongiques activées.

2. Composition selon la revendication 1, caractérisée en ce qu'elle comporte en outre au moins un glucide.

3. Composition selon l'une des revendications 1 à 2, caractérisée en ce qu'elle comporte plus de 10⁶ spores/ml, et de préférence de 10⁷ à 10¹⁰ spores/ml.

4. Composition selon l'une des revendications 1 à 3, caractérisée en ce que les spores sont de préférence des spores activées de moisissures du genre Penicillium ou Geotrichum.

5. Composition selon l'une des revendication 1 à 4, caractérisée en ce que la phase aqueuse est constituée par un soluté aqueux et/ou un milieu de culture desdits spores.

6. Composition selon l'une des revendications 1 à 5, caractérisée en ce que le composé protéique est une glycoprotéine ou un milieu biologique comportant au moins une protéine et/ou une glycoprotéine.

7. Composition selon l'une des revendications 1 à 6, caractérisé en ce que le polyalcool peut être un hexitol, et est de préférence le glycérol.

8. Composition selon l'une des revendications 1 à 7, caractérisée en ce que le glucide est un ose tels que le glucose, le fructose, le galactose ou un oside, tels le lactose, le maltose, le saccharose, ou les amidons, les maltodextrines et les dextrines, ou le sirops de glucose hydrogénés.

9. Composition selon l'une des revendications 1 à 8, caractérisée en ce qu'elle est constituée de 60 à 95 % en poids, de préférence de 70 à 90 % en poids de ladite phase aqueuse, de 5 à 40 % en poids, de préférence de 10 à 30 % en poids dudit composé protéique, de 0,5 à 4 % en poids, de préférence de 1 à 3 % en poids dudit polyalcool et de 5 à 45 % en poids, de préférence de 10 à 30 % en poids dudit glucide.

10. Composition selon l'une des revendications de 1 à 9, caractérisée en ce qu'elle comporte en outre au moins un agent antioxydant, tels l'acide citrique, l'acide ascorbique ou l'acide glutamique ou les sels alcalins ou alcalino-terreux de ces acides.

11. Composition selon la revendication 10, caractérisée en ce qu'elle comporte de 0,1 à 2 % en poids, et de préférence de 0,4 à 1 % en poids dudit agent antioxydant.

12. Composition selon l'une des revendications 1 à 8 et 10 à 11, caractérisée en ce qu'elle comprend en outre un composé hydrophobe et en ce qu'elle se présente sous la forme d'une émulsion eau dans l'huile.

13. Composition selon l'une la revendication 12, caractérisée en ce que le composé hydrophobe est une huile, et de préférence une huile d'origine animale ou végétale.

14. Composition selon l'une des revendications 12 et 13, caractérisée en ce qu'elle est constituée de 15 à 50 % en poids, de préférence de 25 à 41 % en poids de ladite phase aqueuse, de 35 à 80 % en poids, de préférence de 50 à 70 % en poids dudit composé hydrophobe, de 2 à 8 % en poids, de préférence de 3 à 6 %en poids d'au moins un composé protéique, de 0,5 à 4 % en poids, de préférence de 1 à 3 % en poids d'au moins un polyalcool et de 0 à 3 % en poids d'au moins un glucide.

15. Procédé de préparation d'une composition selon l'une des revendications 1 à 14, caractérisée en ce que l'on mélange les spores activées avec la phase aqueuse, au moins un composé protéique, au moins un polyalcool, au moins un composé hydrophobe et/ou au moins un agent antioxydant

16. Procédé selon la revendication 15, caractérisé en ce que l'on mélange la phase aqueuse contenant les spores avec au moins un composé protéique, au moins un polyalcool et éventuellement au moins un glucide, au moins un composé hydrophobe et/ou au moins un agent antioxydant

17. Procédé de conservation de spores activées, caractérisé en ce que l'on conserve une composition selon rune des revendications 1 à 11 à une température de + 4°C ou on congèle ladite composition à une température de - 5°C à - 80°C, de préférence entre - 5°C et -30°C.

18. Procédé de conservation de spores activées, caractérisée en ce que l'on congèle une composition selon l'une des revendications 12 à 14 à une température comprise entre -30°C et -180°C, de préférence entre -30°C et -80°C.

19. Procédé selon la revendication 18, caractérisé en ce qu'on congèle ladite composition en abaissant la température de 0,1 à 10°C/min, de préférence de 0,1 à 3°C, plus préférentiellement de 0,4 à 1,5°C/min.

20. Utilisation d'une composition selon l'une des revendications 1 à 14 dans l'industrie alimentaire, en particulier en fromagerie et en salaison.

## Patentansprüche

1. Zusammensetzung auf Basis von aktivierten Pilzsporen, ausgewählt aus vorgekeimten und/oder gekeimten Sporen, die durch Animpfen eines Kulturmediums oder eines Keimungsmediums erhalten werden können,
dadurch gekennzeichnet, daß die Zusammensetzung eine wäßrige Phase, die mindestens einen Polyalkohol, mindestens eine Proteinzusammensetzung und die genannten aktivierten Pilz-sporen enthält, umfaßt.

2. Zusammensetzung nach Anspruch 1,
dadurch gekennzeichnet, daß sie darüberhinaus mindestens ein Kohlenhydrat enthält.

3. Zusammensetzung nach einem der Ansprüche 1 bis 2,
dadurch gekennzeichnet, daß sie mehr als 10⁶ Sporen/ml und vorzugsweise 10⁷ bis 10¹⁰ Sporen/ml enthält.

4. Zusammensetzung nach einem der Ansprüche 1 bis 3,
dadurch gekennzeichnet, daß die Sporen vorzugsweise aktivierte Sporen von Schimmelpilzen der Gattung Penicillium oder Geotrichum sind.

5. Zusammensetzung nach einem der Ansprüche 1 bis 4,
dadurch gekennzeichnet, daß die wäßrige Phase aus einem wasserhaltigen gelösten Stoff und/oder einem Kulturmedium dieser Sporen gebildet wird.

6. Zusammensetzung nach einem der Ansprüche 1 bis 5,
dadurch gekennzeichnet, daß die Proteinzusammensetzung ein Glykoprotein oder ein biologisches Medium, das mindestens ein Protein und/oder ein Glykoprotein enthält, ist.

7. Zusammensetzung nach einem der Ansprüche 1 bis 6,
dadurch gekennzeichnet, daß der Polyalkohol ein Hexitol sein kann und vorzugsweise Glycerol ist.

8. Zusammensetzung nach einem der Ansprüche 1 bis 7,
dadurch gekennzeichnet, daß das Kohlenhydrat eine Ose, wie Glukose, Fruktose, Galaktose, oder ein Osid, wie Laktose, Maltose, Saccharose oder die Stärken, die Maltodextrine und die Dextrine oder die hydrierten Glukosesirupe, ist.

9. Zusammensetzung nach einem der Ansprüche 1 bis 8,
dadurch gekennzeichnet, daß sie aus 60 bis 95 Gew.-%, vorzugsweise 70 bis 90 Gew.-% der wäßrigen Phase, 5 bis 40 Gew.-%, vorzugsweise 10 bis 30 Gew.-% der Proteinzusammensetzung, 0,5 bis 4 Gew.-%, vorzugsweise 1 bis 3 Gew.-% des Polyalkohols und aus 5 bis 45 Gew.-%, vorzugsweise 10 bis 30 Gew.-% des Kohlenhydrats gebildet wird.

10. Zusammensetzung nach einem der Ansprüche 1 bis 9,
dadurch gekennzeichnet, daß sie darüberhinaus mindestens ein Antioxidationsmittel, wie Zitronensäure, Ascorbinsäure oder Glutaminsäure oder die Alkali- oder Erdalkalimetallsalze dieser Säuren, enthält.

11. Zusammensetzung nach Anspruch 10,
dadurch gekennzeichnet, daß sie 0,1 bis 2 Gew.-% und vorzugsweise 0,4 bis 1 Gew.-% des Antioxidationsmittels enthält.

12. Zusammensetzung nach einem der Ansprüche 1 bis 8 und 10 bis 11,
dadurch gekennzeichnet, daß sie darüberhinaus eine hydrophobe Zusammensetzung enthält und daß sie in Form einer Emulsion von Wasser in Öl vorliegt.

13. Zusammensetzung nach Anspruch 12,
dadurch gekennzeichnet, daß die hydrophobe Zusammensetzung ein Öl ist und vorzugsweise ein Öl tierischen oder pflanzlichen Ursprungs.

14. Zusammensetzung nach einem der Ansprüche 12 und 13,
dadurch gekennzeichnet, daß sie aus 15 bis 50 Gew.-%, vorzugsweise 25 bis 41 Gew.-% der wäßrigen Phase, 35 bis 80 Gew.-%, vorzugsweise 50 bis 70 Gew.-% der hydrophoben Zusammensetzung, 2 bis 8 Gew.-%, vorzugsweise 3 bis 6 Gew.-% von mindestens einer Proteinzusammensetzung, 0,5 bis 4 Gew.-%, vorzugsweise 1 bis 3 Gew.-% von mindestens einem Polyalkohol und 0 bis 3 Gew.-% von mindestens einem Kohlenhydrat gebildet wird.

15. Verfahren zur Herstellung einer Zusammensetzung nach einem der Ansprüche 1 bis 14, dadurch gekennzeichnet, daß man die aktivierten Sporen mit der wäßrigen Phase, mindestens einer Proteinzusammensetzung, mindestens einem Polyalkohol, mindestens einer hydrophoben Zusammensetzung und/oder mindestens einem Antioxidationsmittel mischt.

16. Verfahren nach Anspruch 15,
dadurch gekennzeichnet, daß man die wäßrige Phase, die die Sporen enthält, mit mindestens einer Proteinzusammensetzung, mindestens einem Polyalkohol und gegebenenfalls mindestens einem Kohlenhydrat, mindestens einer hydrophoben Zusammensetzung und/oder mindestens einem Antioxidationsmittel mischt.

17. Verfahren zur Haltbarmachung aktivierter Sporen,
dadurch gekennzeichnet, daß man eine Zusammensetzung nach einem der Ansprüche 1 bis 11 bei einer Temperatur von +4°C konserviert oder man die Zusammensetzung bei einer Temperatur von -5°C bis -80°C, vorzugsweise zwischen -5°C und -30°C einfriert.

18. Verfahren zur Haltbarmachung aktivierter Sporen,
dadurch gekennzeichnet, daß man eine Zusammensetzung nach einem der Ansprüche 12 bis 14 bei einer Temperatur zwischen -30°C und -180°C, vorzugsweise zwischen -30°C und -80°C einfriert.

19. Verfahren nach Anspruch 18,
dadurch gekennzeichnet, daß man die Zusammensetzung durch Erniedrigen der Temperatur um 0,1 bis 10°C/min, vorzugsweise um 0,1 bis 3°C, noch bevorzugter um 0,4 bis 1,5°C/min einfriert.

20. Verwendung einer Zusammensetzung nach einem der Ansprüche 1 bis 14 in der Lebensmittelindustrie, insbesondere bei der Käserei und beim Pökeln.

## Claims

1. Composition based on activated fungal spores chosen from pregerminated spores and/or germinated spores which can be obtained by inoculating a culture medium or a germinating medium, characterised in that the said composition comprises an aqueous phase containing at least one polyalcohol, at least one protein compound and the said activated fungal spores.

2. Composition according to claim 1, characterised in that it contains, in addition, at least one carbohydrate.

3. Composition according to one of claims 1 to 2, characterised in that it comprises more than 10⁶ spores/ml, and preferably from 10⁷ to 10¹⁰ spores/ml.

4. Composition according to one of claims 1 to 3, characterised in that the spores are preferably activated spores of moulds of the genus Penicillium or Geotrichum.

5. Composition according to one of claims 1 to 4, characterised in that the aqueous phase consists of an aqueous solution and/or of a culture medium of the said spores.

6. Composition according to one of claims 1 to 5, characterised in that the protein compound is a glycoprotein or a biological medium comprising at least one protein and/or one glycoprotein.

7. Composition according to one of claims 1 to 6, characterised in that the polyalcohol may be a hexitol, and is preferably glycerol.

8. Composition according to one of claims 1 to 7, characterised in that the carbohydrate is a glycose such as glucose, fructose, galactose or a glycoside such as lactose, maltose, sucrose, or starches, maltodextrins and dextrins, or hydrogenated glucose syrups.

9. Composition according to one of claims 1 to 8, characterised in that it consists of 60 to 95 % by weight, preferably 70 to 90 % by weight, of the said aqueous phase, of 5 to 40 % by weight, preferably 10 to 30 % by weight, of the said protein compound, of 0.5 to 4 % by weight, preferably 1 to 3 % by weight, of the said polyalcohol and of 5 to 45 % by weight, preferably 10 to 30 % by weight, of the said carbohydrate.

10. Composition according to one of claims 1 to 9, characterised in that it comprises, in addition, at least one antioxidant such as citric acid, ascorbic acid or glutamic acid or the alkali metal or alkaline - earth metal salts of these acids.

11. Composition according to claim 10, characterised in that it comprises 0.1 to 2 % by weight, and preferably 0.4 to 1 % by weight, of the said antioxidant.

12. Composition according to one of claims 1 to 8 and 10 to 11, characterised in that it comprises, in addition, a hydrophobic compound and in that it exists in the form of a water-in-oil emulsion.

13. Composition according to claim 12, characterised in that the hydrophobic compound is an oil, and preferably an oil of animal or plant origin.

14. Composition according to one of claims 12 and 13, characterised in that it consists of 15 to 50 % by weight, preferably 25 to 41 % by weight, of the said aqueous phase, of 35 to 80 % by weight, preferably 50 to 70 % by weight, of the said hydrophobic compound, of 2 to 8 % by weight, preferably 3 to 6 % by weight, of at least one protein compound, of 0.5 to 4 % by weight, preferably 1 to 3 % by weight, of at least one polyalcohol and of 0 to 3 % by weight of at least one carbohydrate.

15. Process for preparing a composition according to one of claims 1 to 14, characterised in that the activated spores are mixed with the aqueous phase, at least one protein compound, at least one polyalcohol, at least one hydrophobic compound and/or at least one antioxidant.

16. Process according to claim 15, characterised in that the aqueous phase containing the spores is mixed with at least one protein compound, at least one polyalcohol and, optionally, at least one carbohydrate, at least one hydrophobic compound and/or at least one antioxidant.

17. Process for preserving activated spores, characterised in that a composition according to one of claims 1 to 11 is preserved at a temperature of + 4°C or the said composition is frozen at a temperature of - 5°C to - 80°C, preferably between - 5°C and - 30°C.

18. Process for preserving activated spores, characterised in that a composition according to one of claims 12 to 14 is frozen at a temperature between - 30°C and - 180°C, preferably between - 30°C and - 80°C.

19. Process according to claim 18, characterised in that the said composition is frozen by lowering the temperature by 0.1 to 10°C/min, preferably by 0.1 to 3°C, more preferably by 0.4 to 1.5°C/min.

20. Use of a composition according to one of claims 1 to 14 in the food industry, in particular in cheese-making and in curing.
